# FASCICULE DE BREVET EUROPEEN

(11) **EP 0 792 178 B1**
(45) Date de publication et mention de la délivrance du brevet: **19.08.1998**
(21) Numéro de dépôt: 95940322.1
(22) Date de dépôt: 16.11.1995
(51) Int. Cl.: A61N 1/30

(54) **DISPOSITIF IONOPHORETIQUE D'ADMINISTRATION TRANSDERMIQUE DE MEDICAMENTS ET ENSEMBLE JETABLE FORMANT PARTIE D'UN TEL DISPOSITIF**
IONOPHORETISCHE VORRICHTUNG ZUR TRANSDERMALEN VERABREICHUNG VON MEDIKAMENTEN UNDENTSPRECHENDE WEGWERFEINHEIT
IONOPHORETIC DEVICE FOR TRANSDERMAL ADMINISTRATION OF MEDICAMENTS, AND DISPOSABLE ASSEMBLY FORMING PART OF SUCH DEVICE

(30) Priorité: 16.11.1994 FR 9413715
(43) Date de publication de la demande: 03.09.1997
(73) Titulaire: LABORATOIRES D'HYGIENE ET DE DIETETIQUE (L.H.D.), 75008 Paris (FR)
(72) Inventeur: MILLOT, Philippe, F-21000 Dijon (FR); TEILLAUD, Eric, F-21240 Talant (FR); LAMOISE, Michel, F-21110 Bessey-les-Citeaux (FR)
(74) Mandataire: Colas, Jean-Pierre
(86) Numéro de dépôt international: FR9501509
(87) Numéro de publication internationale: WO9614896

(56) Documents cités:
- WO-A-90/03825
- WO-A-91/05582
- WO-A-93/09842
- WO-A-93/14813

## Description

La présente invention est relative à un dispositif ionophorétique d'administration transdermique de médicaments et, plus particulièrement, à un tel dispositif comprenant un réservoir d'un principe actif sous forme ionisée présentant deux faces sensiblement parallèles, des première et deuxième électrodes en contact électrique avec une face du réservoir et la peau du patient, respectivement, l'autre face du réservoir étant prévue pour s'appliquer sur une plage de la peau du patient espacée de celle sur laquelle porte la deuxième électrode, et des moyens pour faire passer sélectivement un courant électrique entre ces électrodes, pour assister le passage d'un flux du principe actif sous la peau du patient. La présente invention est aussi relative à un ensemble jetable formant partie d'un tel dispositif.

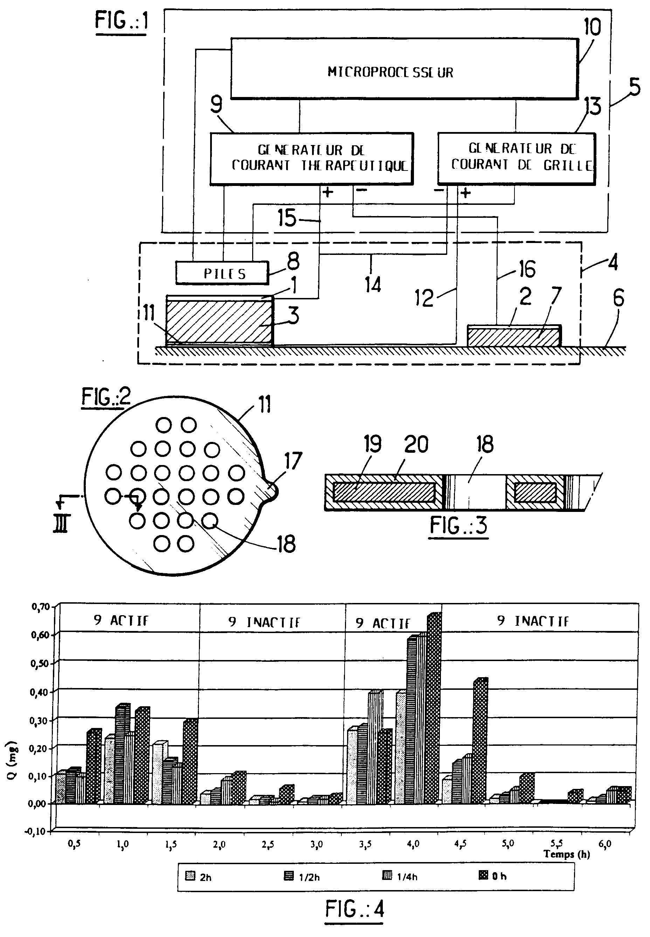

On a schématisé à la figure 1 du dessin annexé un dispositif ionophorétique d'administration transdermique de médicaments comprenant, de manière connue, des première et deuxième électrodes 1,2 respectivement, l'une au moins de ces électrodes (la première sur la figure) étant accolée à un réservoir 3 d'une solution contenant un principe actif sous forme ionisée. A titre d'exemple, ce réservoir 3 peut être constitué par une couche d'un hydrogel imbibé de la solution de principe actif.

Les deux électrodes et le réservoir peuvent être incorporés à un ensemble 4 jetable après usage et conçu pour être associé à un module électronique 5, de manière détachable. Le dispositif constitué par l'ensemble jetable et le module électronique est conçu pour être fixé sur un membre d'un patient de manière que le réservoir 3 s'applique sur la peau 6 de celui-ci, de même que l'électrode 2, sur une plage de la peau adjacente à celle qui reçoit le réservoir. Comme représenté, une couche 7 d'un produit de protection de la peau du patient, tel qu'un hydrogel conducteur de l'électricité, peut être formé sur l'électrode 2 pour s'interposer entre cette électrode et la peau 6 du patient.

Le module électronique 5 est alimenté en énergie électrique par des piles 8 placées de préférence dans l'ensemble jetable 4. Ces piles alimentent ainsi un générateur de courant "thérapeutique" 9 et un microprocesseur 10 programmé pour commander ce générateur.

Lors d'une utilisation de ce dispositif, on charge le réservoir 3 de l'ensemble 4 avec la solution du principe actif à administrer, on installe l'ensemble 4 sur le module électronique 5 et on fixe ceux-ci sur un membre du patient, à l'aide d'un bracelet par exemple, de manière que le réservoir 3 et l'hydrogel 7 s'appliquent contre la peau 6 du patient. On active alors l'alimentation du générateur 9 et du microprocesseur 10 par les piles 8 de manière que ce dernier commande l'établissement d'un courant électrique entre les électrodes 1,2, sous la peau du patient, la polarité du courant étant choisie de manière que les ions du principe actif passent sous la peau du patient à partir du réservoir 3, en descendant le champ électrique ainsi établi.

On comprend qu'il est alors possible de programmer le microprocesseur 10 de manière que celui-ci commande sélectivement l'établissement du courant entre les électrodes, de même que l'intensité de celui-ci. C'est ainsi que, de manière connue, on commande par exemple le passage d'un courant pendant une heure, puis on coupe ce courant pendant les deux heures suivantes, pour le rétablir ensuite, etc... suivant un programme temporel s'étalant ainsi sur six heures ou plus. Les coupures du courant servent en particulier à assurer une "dépolarisation" ainsi qu'un repos physiologique de la peau du patient, de manière à limiter sa dénaturation, dénaturation qui a pour conséquence une altération de ses caractéristiques de perméabilité au principe actif.

On observe que, pendant les périodes où le courant "thérapeutique" est coupé, du principe actif continue de passer sous la peau du patient, sans que l'on puisse contrôler le flux "passif" de principe actif qui s'établit alors. Il s'agit d'un flux osmotique dû au gradient de concentration du principe actif, présent à l'interface entre le réservoir et la peau du patient, gradient qui assure le passage du principe actif sous la peau dans un dispositif transdermique classique, sans assistance ionophorétique. Cette absence de contrôle sur le passage du principe actif pendant les périodes de coupures du courant thérapeutique n'est pas sans inconvénient, notamment lorsque la quantité de principe actif à administrer doit être contrôlée très soigneusement, comme c'est le cas par exemple lorsque celui-ci est un analgésique puissant tel que le fentanyl ou ses dérivés, qui peut être dangereux en cas de dépassement de la dose prescrite.

On connaît de la demande internationale de brevet WO 90/03825 une membrane conçue pour être installée dans un dispositif ionophorétique d'administration transdermique de médicaments, cette membrane réduisant sensiblement le flux de principe actif qui passe sous la peau du patient pendant les périodes de coupures du courant thérapeutique. On observe cependant à la longue, pendant toute la durée d'une administration transdermique de médicaments, une dénaturation progressive de la peau du patient qui accroît progressivement sa perméabilité au principe actif. Avec la membrane décrite au brevet précité, on ne dispose d'aucun moyen permettant de corriger cette croissance temporelle du flux passif, par une action sur la perméabilité de ladite membrane.

La présente invention a pour but de fournir un dispositif ionophorétique d'administration transdermique de médicaments conçu pour réduire ou arrêter le passage d'un flux passif de principe actif pendant les périodes de dépolarisation de la peau du patient et pour corriger toute dérive de la perméabilité de celle-ci due à une dénaturation progressive.

On atteint ces buts de l'invention, ainsi que d'autres qui apparaîtront dans la suite de la description qui va suivre, avec un dispositif ionophorétique d'administration transdermique de médicaments du type décrit en préambule de la présente description, remarquable en ce qu'il comprend en outre a) une grille de commande en matériau conducteur de l'électricité, disposée sur la face du réservoir prévue pour s'appliquer sur la peau du patient et b) des moyens de commande pour couper le courant thérapeutique pendant un intervalle de temps prédéterminé et pour faire passer alors un courant de sens inverse entre la grille et la première électrode, propre à réduire ou arrêter sensiblement le flux passif d'ions du principe actif qui passerait alors autrement sous la peau du patient.

Grâce à la grille de commande du dispositif suivant l'invention, il est non seulement possible de contrôler le flux passif de principe actif quand le courant thérapeutique est coupé, mais il est aussi possible de corriger les effets d'une éventuelle dénaturation de la peau du patient au cours du traitement, comme on le verra dans la suite de la présente description.

Dans un mode de réalisation préféré du dispositif suivant l'invention, les moyens de commande établissent un courant inverse entre la grille et la première électrode pendant une fraction de l'intervalle de temps prédéterminé, à partir du début de cet intervalle de temps. On facilite ainsi la reprise du passage du flux du principe actif au terme de l'intervalle de temps pendant lequel on inhibe ce passage, comme on le verra dans la suite.

De manière commode, le dispositif selon l'invention comprend un ensemble amovible et jetable après usage constitué par les première et deuxième électrodes, au moins un réservoir accolé à une électrode et propre à recevoir une solution de principe actif, au moins une grille de commande collée à ce réservoir et une source d'énergie électrique fournissant le courant d'assistance, le courant inverse et l'alimentation électrique des moyens de commande de ces courants.

D'autres caractéristiques et avantages de la présente invention apparaîtront à la lecture de la description qui va suivre et à l'examen du dessin annexé dans lequel :
- la figure 1 est un schéma du dispositif suivant l'invention, déjà partiellement décrit dans le préambule de la présente description,
- la figure 2 est une vue de dessus d'une grille de commande incorporée au dispositif suivant l'invention,
- la figure 3 est une vue en coupe, schématique, partielle et agrandie, d'un mode de réalisation de la grille de la figure 2, prise suivant le trait de coupe III-III de cette figure, et
- la figure 4 est un diagramme temporel des flux de principe actif observés dans des essais in vitro destinés à mesurer l'efficacité du dispositif suivant l'invention.

On revient à la figure 1 du dessin annexé où il apparaît que le dispositif suivant l'invention comprend une grille de commande 11 placée contre la face du réservoir 3 destinée à être appliquée contre la peau 6 d'un patient. La grille est en un matériau conducteur de l'électricité. La peau du patient est éventuellement protégée d'un contact direct avec cette grille par une mince couche d'un hydrogel tel que celui constituant la couche 7 plaquée contre la deuxième électrode 2.

La grille 11 est reliée par une ligne 12 à un générateur de courant de grille 13 également raccordé à la première électrode par une deuxième ligne 14. Le microprocesseur 10 constitue des moyens de commande sélective des deux générateurs 9,13. C'est ainsi que ce microprocesseur est dûment programmé pour assurer l'exécution d'un programme d'administration du médicament contenu dans le réservoir 3, en commandant le passage d'un courant d'une ligne 15 à une ligne 16, raccordant respectivement les première et deuxième électrodes aux deux sorties du générateur 9, par l'intermédiaire de la partie sous-cutanée du corps du patient comprise entre ces électrodes, puis en coupant cette administration par désactivation du générateur 9, notamment, pendant un intervalle de temps prédéterminé.

Suivant la présente invention, le microprocesseur 10 active le générateur 13 au début de cet intervalle de temps prédéterminé. Un courant de sens inverse de celui du courant thérapeutique précédemment établi circule alors entre la grille de commande 11 et la première électrode 1.

On comprend que, lorsque le générateur 9 est activé par le microprocesseur 10, la grille 11 n'est pas alimentée en courant. Elle n'oppose alors au passage du principe actif qu'un effet mécanique d'écran, minimisé par une structure ajourée convenable. L'administration transdermique de médicaments sous assistance ionophorétique s'accomplit alors normalement.

Par contre, quand le microprocesseur 10 active le générateur 13 et coupe le générateur 9, la grille 11 devient anode et l'électrode 1, cathode. Si le principe actif contenu dans le réservoir est cationique, les cations qui le constituent vont alors se diriger vers la première électrode, devenue cathode. Le flux de cations ainsi établi s'oppose alors au flux "passif" des cations du principe actif que l'on observe autrement en transdermie classique, sans assistance ionophorétique. En arrêtant ou en freinant ainsi efficacement le flux de principe actif pendant les intervalles de temps d'un programme d'administration où l'on souhaite arrêter ce flux, on assure d'une part une meilleure "dépolarisation" de la peau du patient et, d'autre part, un contrôle plus rigoureux des quantités de principe actif effectivement administré à celui-ci.

On a représenté à la figure 2 une géométrie possible pour la grille de commande 11 à accoler au réservoir 3 de principe actif. Celle-ci est percée d'ouvertures 18 régulièrement distribuées et destinées à assurer le passage d'un flux de principe activé ionophorétiquement quand le générateur 9 débite du courant entre les électrodes 1 et 2. Comme on l'a vu plus haut, la grille peut être réalisée à partir d'une mince feuille (de 25 µm d'épaisseur par exemple) d'un métal conducteur tel que l'argent, le cuivre, le zinc ou l'or.

Le principe actif est souvent introduit dans une solution de chlorure de sodium ou de potassium, par exemple, et c'est la solution qui imprègne le réservoir 3. Quand le générateur 13 de courant de grille est activé, et si la grille (alors "anode") est constituée en simple métal, les ions Cl⁻ de la solution se plaquent sur la grille, ce qui provoque un appauvrissement de la solution en ions Cl⁻.

Pour éviter un abaissement de la conductivité du réservoir et une électrolyse de l'eau qui augmenterait le pH de la solution avec pour conséquence une irritation de la peau du patient, il est préférable d'utiliser une grille de commande en argent plaqué électrochimiquement de AgCl, ce qui évite de consommer les ions Cl⁻ de la solution quand la grille est alimentée. La figure 3 du dessin annexé représente un agrandissement de la coupe partielle, suivant le trait de coupe III-III de la figure 2, faisant apparaître une feuille ajourée 19 en argent, recouverte d'un placage 20 de chlorure d'argent. Les première et deuxième électrodes peuvent d'ailleurs être constituées de même.

La grille de commande 11 pourrait encore être constituée par un film ajouré en polymère recouvert de carbone, ou, par sérigraphie, d'une encre conductrice.

L'efficacité du dispositif suivant l'invention est illustrée par le diagramme temporel de la figure 4, établi à l'aide de cellules de mesures "in vitro" comprenant chacune une enceinte pour un milieu receveur des ions d'un principe actif, en l'occurrence le chlorhydrate de morphine, ayant traversé un échantillon de peau accolée au réservoir du dispositif de la figure 1. L'électrode 2 de ce dispositif trempe alors dans le milieu receveur pour assurer la circulation du courant électrique à travers la peau. En dosant périodiquement la concentration en principe actif du milieu receveur, on peut calculer la quantité Q (en mg) de principe actif ayant franchi la peau pendant un intervalle de temps prédéterminé, soit sous courant thérapeutique (générateur 9 actif) soit pendant une période de repos (générateur 9 inactif).

Le programme de traitement illustré par la figure 4 dure 6 heures et comprend deux périodes d'administration du médicament sous courant thérapeutique, de 1 heure, suivie chacune d'une période de repos (générateur 9 inactif) de 2 heures. Les courants continus délivrés par les générateurs 9 et 13 sont de 1mA et 8mA respectivement.

Avantageusement, le générateur 13 peut comprendre une alimentation à découpage capable de délivrer ce courant de 8mA, sous 40 à 60 volts par exemple, à partir de la tension de quelques volts délivrée par les piles 8.

On a étudié l'influence de la durée de l'alimentation de la grille de commande 11 du dispositif suivant l'invention, pendant les périodes de repos. C'est ainsi que le diagramme de la figure 4 rassemble les valeurs moyennes des mesures obtenues avec quatre groupes de cellules dont les grilles de commande ont été alimentées par le générateur 13 pendant deux heures, 1/2 heure, 1/4 d'heure et 0 heure respectivement, pendant les périodes de repos, à partir du début de ces périodes. Le graphe permet de mesurer l'efficacité de la grille de commande du dispositif suivant l'invention et l'influence de la durée de son alimentation en période de repos.

On remarque une certaine dispersion des quantités de principe actif ayant franchi les échantillons de peau pendant la première heure sous courant thérapeutique, alors même que les quatre groupes de cellules concernés comprennent des échantillons de peau qui n'ont pas encore connu une période d'activation de la grille de commande 11. Cette dispersion s'explique par l'inévitable dispersion des caractéristiques des échantillons de peau placés dans les cellules. On observe simplement que la présence de la grille de commande, par son effet mécanique d'écran, à tendance à réduire le flux de principe actif.

Pendant les deux heures qui suivent la fin de ce premier intervalle de temps d'une heure, le courant thérapeutique est coupé et la grille 11 alimentée. On observe une forte réduction des quantités de principe actif qui franchissent alors les échantillons de peau, par rapport à la quantité résultant du seul flux "passif" (pas d'alimentation de la grille 11). C'est la preuve de l'efficacité de ladite grille dans son rôle de blocage ou de réduction de ce flux passif. La présente invention permet ainsi de commander plus précisément les quantités de principe actif transmises au patient, en réduisant ou en supprimant l'influence du flux "passif" pendant les nécessaires périodes de repos de la peau, où le courant thérapeutique est supprimé. Il est même possible, suivant l'invention, de réduire de manière contrôlée ce flux passif en faisant varier par exemple le courant délivré par le générateur 13 de courant de grille pendant les périodes de coupure du générateur 9. L'invention offre alors un contrôle constant du flux de principe actif aussi bien sous courant thérapeutique qu'en l'absence d'un tel courant.

A la reprise du courant thérapeutique (quatrième heure du traitement) on observe que les quantités de principe actif traversant la peau sont fortement accrues par rapport à celles observées pendant la première heure. Cela est dû à la dénaturation de la peau due au courant et à l'effet occlusif des électrodes, qui se sont opérés pendant les trois heures précédentes, dénaturation qui a pour effet d'accroître la perméabilité de la peau au principe actif, et ce malgré la modération de cet accroissement dû à la période de dépolarisation intermédiaire de deux heures.

On observe que la reprise du passage du principe actif est d'autant plus forte que la durée d'application d'un courant de blocage à la grille 11 pendant la période de repos antérieure est plus courte. Cette observation peut être utilisée pour accélérer ou retarder l'administration de la quantité totale de principe actif nécessaire au patient en fonction de considérations pharmacologiques. Pendant la période de repos de deux heures qui suit la deuxième application du courant thérapeutique aux électrodes 1 et 2 par le générateur 9, la réalimentation de la grille de commande 11 abaisse encore fortement le flux passif de principe actif à travers la peau, par rapport à celui que l'on observe en l'absence d'alimentation de la grille de commande.

On a repris les essais décrits ci-dessus en remplaçant les courants continus utilisés par des courants pulsés à la fréquence de 500 Hz avec un rapport cyclique de 50% pour améliorer la dépolarisation de la peau. Les résultats observés en ce qui concerne le contrôle du flux passif de principe actif sont du même ordre et confirment ceux obtenus en courant continu.

Il apparaît ainsi que l'invention permet bien d'atteindre le but fixé, soit une forte réduction du flux "passif" observé lorsqu'on coupe le courant thérapeutique. Avantageusement, le microprocesseur 10 peut être équipé de moyens pour faire varier la durée d'application du courant délivré par le générateur 13 à la grille de commande, de manière à autoriser une action sur la "reprise" ultérieure du flux de principe actif comme on l'a vu plus haut.

Le microprocesseur 10 peut aussi être équipé de moyens permettant de faire varier l'intensité du courant d'alimentation de la grille de commande, de manière à corriger avantageusement toute dérive temporelle de la perméabilité de la peau du patient au principe actif.

Avantageusement encore, le dispositif suivant l'invention peut être équipé de moyens sensibles à la tension établie entre la première électrode 1 et la grille de commande 11 et à l'intensité du courant circulant entre elles pour en déduire une mesure de la quantité de principe actif demeurant dans le réservoir 3, suivant le procédé décrit dans la demande de brevet français déposée le 6 mai 1994 par la demanderesse et intitulée "Procédé et dispositif de mesure de la quantité de principe actif contenu dans un réservoir".

Bien entendu l'invention n'est pas limitée au mode de réalisation décrit et représenté qui n'a été donné qu'à titre d'exemple. Ainsi, le dispositif peut comprendre un deuxième réservoir de principe actif et une deuxième grille de commande associée à la deuxième électrode 2 pour l'établissement sélectif d'un flux de principe actif à partir de ce deuxième réservoir et pour le blocage sélectif du flux passif inverse en période de coupure de courant d'assistance passant de la deuxième électrode à la première électrode, par dessous la peau du patient.

On a vu plus haut que la grille de commande peut être constituée en un métal identique à celui des électrodes 1 et 2. En variante, on pourrait choisir un autre métal pour constituer la grille de commande, de manière à établir un couple électrochimique entre cette grille et l'électrode 1, propre à renforcer le blocage des ions du principe actif en période de repos, sans pour autant réduire sensiblement l'intensité du courant thérapeutique pendant les périodes d'administration du principe actif.

De même, l'invention est également applicable à un dispositif transdermique classique dépourvu de moyens de fourniture d'un courant thérapeutique d'assistance. Sa mise en oeuvre est cependant particulièrement commode dans un dispositif d'administration transdermique sous assistance ionophorétique du fait de la présence d'électrodes nécessaires au passage du courant thérapeutique ainsi qu'au courant inverse suivant la présente invention.

Egalement, l'invention s'étend à l'administration du chlorydrate ou autre sel de métoclopramide ou de mélatonine et, plus généralement, à l'administration de tout principe actif.

## Revendications

1. Dispositif ionophorétique d'administration transdermique de médicaments, comprenant un réservoir (3) d'un principe actif sous forme ionisé présentant deux faces sensiblement parallèles, des première (1) et deuxième (2) électrodes en contact électrique avec une face du réservoir (3) et la peau (6) du patient, respectivement, l'autre face du réservoir étant prévue pour s'appliquer sur une plage de la peau (6) du patient espacée de celle sur laquelle porte la deuxième électrode et des moyens (5) pour faire passer sélectivement un courant électrique entre ces électrodes, pour assister le passage d'un flux du principe actif sous la peau du patient, dispositif caractérisé en ce qu'il comprend en outre a) une grille de commande (11) en matériau conducteur de l'électricité, disposée sur la face du réservoir (3) prévue pour s'appliquer sur la peau (6) du patient et b) des moyens de commande (10,9,13) pour couper le courant d'assistance pendant un intervalle de temps prédéterminé et pour faire passer alors un courant de sens inverse entre la grille (11) et la première électrode (1), propre à réduire ou arrêter sensiblement le flux passif d'ions du principe actif qui passerait alors autrement sous la peau du patient.

2. Dispositif conforme à la revendication 1, caractérisé en ce que lesdits moyens de commande (10,13) établissent un courant inverse entre la grille (11) et la première électrode (1) pendant une fraction de l'intervalle de temps prédéterminé, à partir du début de cet intervalle de temps.

3. Dispositif conforme à la revendication 2, caractérisé en ce que lesdits moyens de commande (10) comprennent des moyens pour faire varier la durée de ladite fraction de l'intervalle de temps prédéterminé et des moyens pour faire varier l'intensité du courant inverse alors appliqué.

4. Dispositif conforme à l'une quelconque des revendications 1 à 3, caractérisé en ce qu'il comprend des moyens sensibles à la tension établie entre la première électrode (1) et la grille (11) de commande et à l'intensité du courant circulant entre elles pour en déduire une mesure de la quantité de principe actif demeurant dans le réservoir.

5. Dispositif conforme à l'une quelconque des revendications 1 à 4, caractérisé en ce qu'un deuxième réservoir de principe actif et une deuxième grille de commande sont associés à la deuxième électrode (2) pour l'établissement sélectif d'un flux de principe actif à partir de ce deuxième réservoir et pour le blocage sélectif du flux passif en période de coupure d'un courant d'assistance passant de la deuxième électrode à la première électrode, par dessous la peau du patient.

6. Dispositif conforme à l'une quelconque des revendications 1 à 5, caractérisé en ce que la grille de commande est constituée d'un métal conducteur.

7. Dispositif conforme à la revendication 6, caractérisé en ce que ledit métal conducteur est choisi pour former un couple électrochimique avec celui constituant la première électrode (1), propre à renforcer le blocage des ions du principe actif en période de repos.

8. Dispositif conforme à la revendication 6, caractérisé en ce qu'il comprend une membrane échangeuse d'ions placée contre la grille pour empêcher une migration d'ions métalliques vers la peau du patient.

9. Dispositif conforme à l'une quelconque des revendications 1 à 5, dans lequel le principe actif est introduit dans le réservoir par l'intermédiaire d'une solution de NaCl, caractérisé en ce que la grille est constituée d'argent métal plaqué d'ions Cl⁻.

10. Dispositif conforme à l'une quelconque des revendications 1 à 5, caractérisé en ce que la grille est constituée par un film ajouré d'un matériau polymère, recouvert d'une matière conductrice.

11. Ensemble amovible jetable après usage, formant partie du dispositif conforme à l'une quelconque des revendications 1 à 10, caractérisé en ce qu'il comprend les première (1) et deuxième (2) électrodes, au moins un réservoir (3) accolé à une électrode et propre à recevoir une solution de principe actif, au moins une grille de commande (11) accolée à ce réservoir et une source d'énergie électrique (8) fournissant le courant d'assistance, le courant inverse et l'alimentation électrique des moyens de commande (10) de ces courants.

## Claims

1. Iontophoretic device for transdermal delivery of medicinal products, comprising a reservoir (3) for an active principle in ionized form, having two substantially parallel faces, first (1) and second (2) electrodes in electrical contact with a face of the reservoir (3) and the skin (6) of the patient, respectively, the other face of the reservoir being intended to be applied onto a region of the skin (6) of the patient which lies away from the region on which the second electrode bears, and means (5) for selectively passing an electric current between these electrodes, in order to assist the passage of a flow of the active principle under the skin of the patient, which device is characterized in that it furthermore comprises a) a control grid (11) made of electrically conducting material, arranged on the face of the reservoir (3) intended to be applied onto the skin (6) of the patient and b) control means (10, 9, 13) for cutting the assistance current for a predetermined time interval and for then passing a current in the opposite direction between the grid (11) and the first electrode (1), which current can reduce or substantially stop the passive flow of ions of the active principle which would then otherwise pass under the skin of the patient.

2. Device according to Claim 1, characterized in the said control means (10, 13) establish an inverse current between the grid (11) and the first electrode (1) for a fraction of the predetermined time interval, starting from the beginning of this time interval.

3. Device according to Claim 2, characterized in that the said control means (10) comprise means for varying the duration of the said fraction of the predetermined time interval and means for varying the strength of the inverse current then applied.

4. Device according to any one of Claims 1 to 3, characterized in that it comprises means sensitive to the voltage set up between the first electrode (1) and the control grid (11) and to the strength of the current flowing between them for deducing therefrom a measure of the quantity of active principle remaining in the reservoir.

5. Device according to any one of Claims 1 to 4, characterized in that a second reservoir of active principle and a second control grid are associated with the second electrode (2), for selectively establishing a flow of active principle from this second reservoir and for selectively blocking the passive flow in the cut-off period of an assistance current passing from the second electrode to the first electrode, below the skin of the patient.

6. Device according to any one of Claims 1 to 5, characterized in that the control grid consists of a conducting metal.

7. Device according to Claim 6, characterized in that the said conducting metal is chosen to form an electrochemical couple with the metal constituting the first electrode (1), capable of reinforcing blocking of the ions of the active principle in the rest period.

8. Device according to Claim 6, characterized in that it comprises an ion-exchange membrane placed against the grid for preventing migration of metal ions to the skin of the patient.

9. Device according to any one of Claims 1 to 5, in which the active principle is introduced into the reservoir by means of a solution of NaCl, characterized in that the grid consists of metal silver plated with Cl⁻ ions.

10. Device according to any one of Claims 1 to 5, characterized in the grid consists of an open-worked film of a polymer material, covered with a conductive material.

11. Disposable removable assembly, forming part of the device according to any one of Claims 1 to 10, characterized in that it comprises first (1) and second (2) electrodes, at least one reservoir (3), attached to one electrode and capable of holding a solution of active principle, at least one control grid (11) attached to this reservoir and an electrical power source (8) supplying the assistance current, the inverse current and the electrical supply of the means (10) for controlling these currents.

## Patentansprüche

1. Iontophoresevorrichtung zur transdermalen Verabreichung von Arzneimitteln mit einem Behälter (3) mit zwei im wesentlichen parallelen Seiten für einen Wirkstoff in ionisierter Form, mit einer ersten Elektrode (1) und einer zweiten Elektrode (2), die mit einer Seite des Behälters (3) beziehungsweise mit der Haut (6) des Patienten in Kontakt ist, wobei die andere Seite des Behälters dafür vorgesehen ist, auf einen Bereich der Haut (6) des Patienten aufgelegt zu werden, der von dem Bereich entfernt ist, auf welchem die zweite Elektrode aufliegt, sowie mit Mitteln (5), um zwischen diesen Elektroden selektiv einen Strom fließen zu lassen, um das Eintreten eines Wirkstoffflusses unter die Haut des Patienten zu unterstützen, dadurch gekennzeichnet, daß sie außerdem a) ein Steuergitter (11) aus elektrisch leitendem Werkstoff, das auf der zum Auflegen auf die Haut (6) des Patienten vorgesehenen Seite des Behälters (3) angeordnet ist, und b) Steuereinrichtungen (10, 9, 13) aufweist, um den Unterstützungsstrom während eines vorbestimmten Zeitintervalls zu unterbrechen und um dann einen Strom in umgekehrter Richtung zwischen dem Gitter (11) und der ersten Elektrode (1) fließen zu lassen, welcher geeignet ist, den passiven Fluß von Wirkstoffionen zu verringern oder zu unterbrechen, der andernfalls unter die Haut des Patienten gelangen würde.

2. Vorrichtung nach Anspruch 1, dadurch gekennzeichnet, daß die Steuereinrichtungen (10, 13) einen umgekehrten Strom zwischen dem Gitter (11) und der ersten Elektrode (1) während eines Teils des vorbestimmten Zeitintervalls und vom Anfang dieses Zeitintervalls an herstellen.

3. Vorrichtung nach Anspruch 2, dadurch gekennzeichnet, daß die Steuereinrichtungen (10) Einrichtungen aufweisen, um die Dauer dieses Teils des vorbestimmten Zeitintervalls zu ändern, sowie Einrichtungen, um die Stärke des dabei angelegten umgekehrten Stroms zu ändern.

4. Vorrichtung nach einem der Ansprüche 1 bis 3, dadurch gekennzeichnet, daß sie Einrichtungen aufweist, die gegenüber der zwischen der ersten Elektrode (1) und dem Steuergitter (11) hergestellten Spannung und gegenüber der Stärke des zwischen diesen fließenden Stroms empfindlich sind, um daraus eine Messung der noch im Behälter verbleibenden Wirkstoffmenge abzuleiten.

5. Vorrichtung nach einem der Ansprüche 1 bis 4, dadurch gekennzeichnet, daß ein zweiter Wirkstoffbehälter und ein zweites Steuergitter der zweiten Elektrode (2) zugeordnet sind, und zwar für die selektive Erzeugung eines Wirkstoffflusses von diesem zweiten Behälter aus und für die selektive Blockierung des passiven Flusses in der Periode der Unterbrechung eines Unterstützungsstroms, der von der zweiten Elektrode unter der Haut des Patienten zur ersten Elektrode fließt.

6. Vorrichtung nach einem der Ansprüche 1 bis 5, dadurch gekennzeichnet, daß das Steuergitter aus einem leitenden Metall besteht.

7. Vorrichtung nach Anspruch 6, dadurch gekennzeichnet, daß dieses leitende Metall ausgewählt ist, um mit demjenigen, das die erste Elektrode (1) bildet, ein elektrochemisches Paar zu bilden, das geeignet ist, die Blockierung der Ionen des Wirkstoffs in einer Ruheperiode zu verstärken.

8. Vorrichtung nach Anspruch 6, dadurch gekennzeichnet, daß sie eine auf dem Gitter angeordnete Ionenaustauschmembran aufweist, um ein Wandern von Metallionen auf die Haut des Patienten zu zu verhindern.

9. Vorrichtung nach einem der Ansprüche 1 bis 5, in welcher der Wirkstoff in den Behälter über eine NaCl-Lösung eingebracht wird, dadurch gekennzeichnet, daß das Gitter aus mit Cl⁻-Ionen beschichtetem Metallsilber besteht.

10. Vorrichtung nach einem der Ansprüche 1 bis 5, dadurch gekennzeichnet, daß das Gitter aus einer durchbrochenen Folie aus einem Polymermaterial besteht, die mit einem leitenden Werkstoff überzogen ist.

11. Abnehmbare,nach Gebrauch wegwerfbare Einheit, die einen Teil der Vorrichtung nach einem der Ansprüche 1 bis 10 bildet, dadurch gekennzeichnet, daß sie die erste Elektrode (1) und die zweite Elektrode (2), mindestens einen an eine Elektrode angefügten Behälter (3), der eine Wirkstofflösung aufnehmen kann, mindestens ein an den Behälter angefügtes Steuergitter (11) und eine elektrische Energiequelle (8) aufweist, die den Unterstützungsstrom, den umgekehrten Strom und die elektrische Versorgung der Einrichtungen (10) zur Steuerung dieser Ströme liefert.
